# EUROPEAN PATENT SPECIFICATION

(11) **EP 0 647 157 B1**
(45) Date of publication and mention of the grant of the patent: **25.09.1996**
(21) Application number: 93916629.4
(22) Date of filing: 18.06.1993
(51) Int. Cl.: B01J 23/96, B01J 38/10, C07C 7/167

(54) **REGENERATION OF ACETYLENE CONVERTER CATALYSTS BY HYDROGEN STRIPPING**
REGENERIERUNG VON ACETYLEN-UMWANDLER-KATALYSATOREN DURCH SPÜLEN MIT WASSERSTOFF
REGENERATION DE CATALYSEURS DE CONVERSION D'ACETYLENE PAR EXTRACTION PAR HYDROGENE

(30) Priority: 19.06.1992 US 901321
(43) Date of publication of application: 12.04.1995
(73) Proprietor: EXXON CHEMICAL PATENTS INC., Baytown, TX 77520-2149 (US)
(72) Inventor: HUANG, Yao-Jyh, Robert, Houston, TX 77062 (US); FUNG, Chun, Chong, Bridgewater, NJ 08807 (US); DANIEL, Lawrence, Gilbert, Crosby, TX 77532 (US); MOHUNDRO, Edgar, Lucian, Baytown, TX 77521 (US); HARTGERINK, John, Edward, Baton Rouge, LA 70810 (US)
(74) Representative: Bawden, Peter Charles
(86) International application number: PCT/US93/05880
(87) International publication number: WO 94/00232

(56) References cited:
- DE-A- 3 022 043
- DE-A- 4 022 852
- GB-A- 1 048 343
- US-A- 3 893 942

## Description

### BACKGROUND OF THE INVENTION

### Field of the Invention

The present invention relates to an improved method for regenerating catalysts that have been used to remove acetylenic contaminants from olefin product streams. More particularly, the present invention relates to an improved method for regenerating palladium-containing catalysts that have been used to remove acetylenic contaminants from ethylene.

Product streams of liquid or liquefiable olefins and diolefins typically are contaminated with small amounts of acetylenic impurities. For example, the ethylene/ethane fraction in an ethylene plant typically contains 0.5 - 2.0% acetylenic impurities. Such acetylenic contamination is undesirable because acetylene tends to poison the catalysts that are used to convert ethylene to polyethylene. Acetylene also can form metal acetylides which are explosive and which may contaminate the equipment that is used to process such product streams.

It is known that acetylenic impurities can be selectively hydrogenated and thereby removed from such a product streams by passing the product stream over a catalyst (an "acetylene hydrogenation catalyst") in the presence of hydrogen; however, the hydrogenation process typically results in the deposition of a green oil on the catalyst which deactivates the catalyst. Therefore, acetylene hydrogenation processes typically include an oxygenation step or a "burn" step to remove the deactivating carbonaceous residues from the catalyst followed by a hydrogen reduction step to reactivate the catalyst. See, e.g., US-A-3,812,057 (Morgan) and US-A-4,424,255 (Toyoda).

An oxygenation step followed by hydrogen reduction normally is effective to regenerate an acetylene hydrogenation catalyst; however, there are many disadvantages to the use of an oxygenation step during regeneration of such catalysts.

First, when oxygen and hydrogen come together under the conditions used to regenerate the catalyst, an exothermic reaction is produced which may result in uncontrolled temperature runaway. Thus, before the regeneration is performed, it is necessary to purge the catalyst of hydrogen and hydrocarbons using an inert gas, such as nitrogen gas, both before and after the oxygen-containing stream is introduced. The purging process is both tedious and time consuming. Furthermore, if the regeneration is performed in situ, the concentration of oxygen in the regeneration gas stream must be regulated carefully in order to avoid reactor temperature run-away. It would be advantageous if acetylene hydrogenation catalysts could be regenerated using a method which did not require such time consuming purging and monitoring.

The use of an oxygenation step during regeneration of an acetylene hydrogenation catalyst also presents an environmental concern due to the resulting emission of carbon monoxide, carbon dioxide, and unburned hydrocarbons. The hydrocarbon residue that remains on the catalyst can comprise up to 10 wt% of the catalyst. The oxygenation of this amount of residue results in the emission of a substantial amount of carbon monoxide and carbon dioxide. In addition, the emission during oxygenation may contain approximately 6-10% of unburned hydrocarbons. It would be advantageous if acetylene reduction catalysts could be regenerated in a manner that did not result in such environmentally undesirable emissions.

Regeneration of acetylene hydrogenation catalysts using an oxygenation step also is undesirable because the oxygenation step has a negative impact on the life of the catalyst. A catalyst that has been used to hydrogenate acetylene typically must be regenerated once every one to three months. Acetylene hydrogenation catalysts are heat sensitive, and exposure to excessive heat decreases the activity of the catalyst. The oxygenation step that is used in most regeneration processes typically is run at temperatures between 371-455°C (700-850°F). The exposure of an acetylene hydrogenation catalyst to such high temperatures at such regular intervals takes a great toll on the life of the catalyst. It would be advantageous if the regeneration of such catalysts could take place at lower temperatures which reduced the thermal stress induced during the regeneration.

Some of these disadvantages can be avoided if the catalyst is regenerated ex situ, or outside of the reactor. However, the unloading and reloading of the reactor with the catalyst is a time consuming process. Even if the regeneration process is performed ex situ, the catalyst still must be purged with an inert gas both before unloading and after reloading the reactor. Thus, regardless of where the regeneration process takes place, significant reactor down time is required if the catalyst is regenerated using a process that includes an oxidation step.

For all of these reasons, it would be advantageous if acetylene reducing catalysts could be regenerated without an oxygenation step. Col. 4, lines 16-35 of US-A-3,912,789 acknowledges that partial regeneration can be accomplished using a hydrogenation step alone. However, this patent teaches that full regeneration of an exhausted catalyst requires an oxygenation step. Therefore, a process has yet to be developed that can fully regenerate acetylene reduction catalysts without the need for an oxygenation step.

Not only is an oxygenation step undesirable, but any additional step in the regeneration procedure is undesirable because each step requires a change of conditions and switch over techniques which can be deleterious to the performance or longevity of the regenerated catalyst. For example, GB-1,158,418 teaches a method of regenerating hydrogenation catalysts comprising a hydrocarbon wash step followed by a hydrogen treatment. Indeed this patent discusses an already known method consisting of contacting hydrogen with the catalyst at an elevated temperature (p. 1, line 77 - p. 2, line 10), but reports that this known method was still insufficient to restore completely the initial activity of the selective hydrogenation catalyst.

US-A-2,946,829 discloses a process of making a Pd/Al₂O₃ catalyst for selective hydrogenation, used with the industry standard method of regeneration, which uses steam to provide heat to attain the needed temperature to burn the polymeric deposits off the catalyst. When a small amount of air is mixed into the steam (see Example 3, col. 5, 1. 52-55), the resulting catalyst is relatively free of carbonaceous deposits. The main function of the hydrogen reduction step is to reduce the oxidized metal back to its metallic state. In fact, the hydrogen reduction step is not even required, as shown in Example 3, col. 5, 1. 65. On day 17, regeneration 6 did not include a reduction step. The catalyst nevertheless was put back on stream after steam treatment and exhibited very high activity, i.e., at a space velocity of 2,000 and 66°C (150°F), acetylene in the reactor outlet dropped from an inlet concentration of 0.35% to 1.3 ppm. This compares favorably to the results of the first regeneration, Example 1, which did use a hydrogen reduction step after steam treatment -- 5 ppm acetylene at the reactor outlet at a much lower space velocity of 1,000 and 54°C (130°F).

In addition, US-A-2,946,829 teaches the need for vigorous regeneration in order to remove polymer deposits. Such severe regeneration leads to a loss of physical strength, in the hydrogenation catalyst, or to impaired activity or selectivity (col. 2, lines 30-37). The vigorous step in the disclosure is the step of burning off the polymer deposits in the presence of steam -- not the hydrogen reduction step. Thus, this art teaches that the steam treatment step is essential to regeneration of a hydrogenation catalyst.

GB-A-907,348 teaches the partial regeneration of a nickel catalyst using a hydrogen treatment. Although a temperature range of 200-600°C is mentioned, all of the actual regenerations described are carried out at temperatures in the range of 204-250°C. In the Provisional Specification of GB-A-907,348, temperatures at the lower end of the range are said to be preferred, particularly 200-300°C. From the actual experiments of GB-A-907,348, regeneration at 250°C did not cause recovery of fresh catalyst activity, and the stability of the regenerated catalyst was lower except in the experiment that used the lowest temperature (204°C). (See Examples 1-3.) The failure to recover the activity of fresh catalyst is clear because a higher temperature is required to maintain the same hydrogen consumption during regeneration (Example 1, 127°C fresh v. 143°C regenerated; Example 2, 127°C fresh v. 138°C regenerated). And, in Examples 1 and 2 (which used a regeneration temperature of 250°C), the stability of the regenerated catalyst is lower (Example 1, fresh catalyst required 152°C after 670 hours v. regenerated catalyst required 166°C after 477 hours; Example 2, fresh catalyst required 127°C after 335 hours v. regenerated catalyst required 138°C after 127 hours). However, in Example 3 (which regenerates the catalyst at a temperature of 204°C), although the activity of the fresh catalyst is not completely recovered, the stability of the regenerated catalyst is higher than the stability of the fresh catalyst (regenerated catalyst required 142°C after 1410 hours, fresh catalyst required 160°C after 327 hours). Thus this publication teaches away from the regeneration of catalysts using hydrogen at temperatures that are above 250°C.

### SUMMARY OF THE INVENTION

According to the present invention there is provided a method of substantially completely regenerating a catalyst that has been used to remove acetylenic impurities from an olefin stream and which has become at least partially deactivated by that use, which comprises substantially stripping the deactivated catalyst of carbonaceous deposits using hydrogen at a linear velocity of at least 15.2 cm/second (0.5 feet per second), at a temperature of from 315 - 400°C (599 - 750°F) and at other conditions such that substantially complete regeneration is effected, wherein said stripping is essentially the only regeneration treatment employed and occurs in the absence of an oxygenation step.

It is a feature of the invention that acetylene reduction catalysts can be substantially fully regenerated by hydrogen stripping, alone, at relatively low temperatures which are nevertheless greater than those taught away from in prior art such as GB-A-907,348. It has also been found that palladium-containing catalysts that have been used to purify ethylene unexpectedly have a better selectivity to ethylene after they are hydrogen stripped according to the present invention.

### BRIEF DESCRIPTION OF THE DRAWINGS

Fig. 1 is a chart showing the Temperature Programmed Oxidation (TPO) spectra of the UCI G58B catalyst of Example 1 before (a) and after (b) hydrogen stripping.

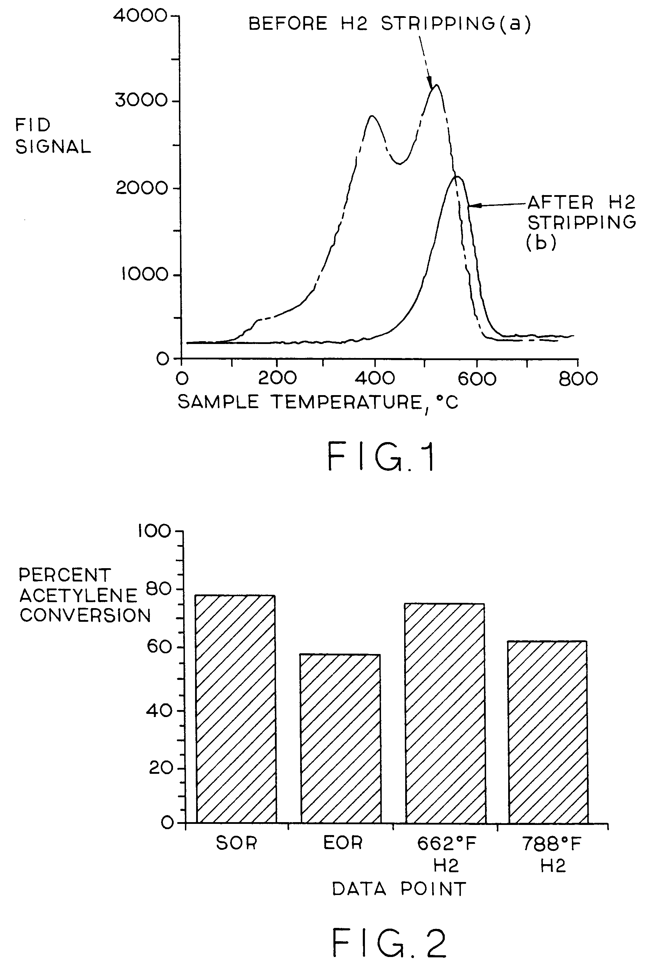

Fig. 2 is a bar graph comparing the catalyst activity of the BASF H011 catalyst of Example 2 before and after hydrogen stripping. "SOR" under the first bar in the graph stands for activity at the start of the run. "EOR" under the second bar in the graph stands for activity at the end of the run. The bar above "662°F H2" represents the activity of the substantially completely regenerated catalyst after hydrogen stripping according to the invention at about 350°C (662°F). The bar above "788°F H2" represents the (reduced) activity of the incompletely regenerated catalyst after further hydrogen stripping outside the scope of the invention at about 420°C (788°F).

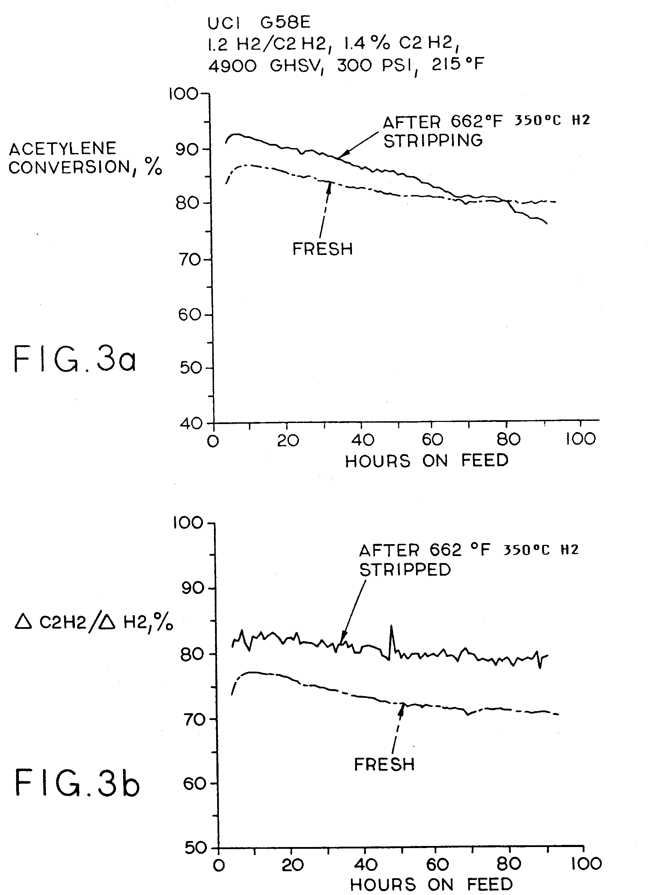

Fig. 3(a) is a graph comparing the acetylene conversion activity of the UCI G58E catalyst of Example 3 when fresh to the activity of that same (substantially completely regenerated) catalyst after it has been hydrogen stripped according to the present invention at about 350°C (662°F).

Fig. 3(b) is a graph comparing the deactivation rate of the fresh and stripped catalyst of Example 3.

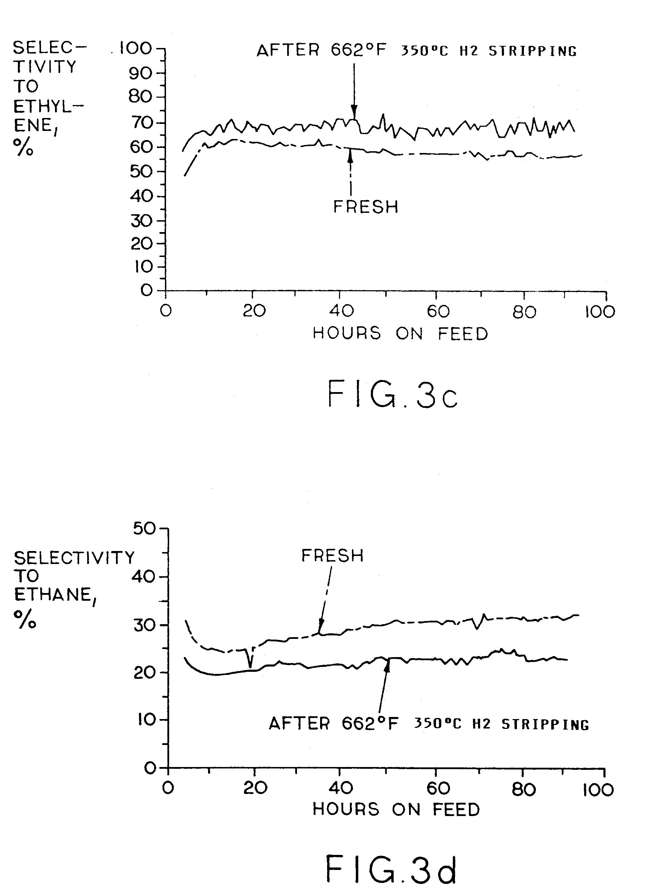

Fig. 3(c) is a graph comparing the selectivity to ethylene of the fresh and stripped catalyst of Example 3.

Fig. 3(d) is a graph comparing the selectivity to ethane of the fresh and stripped catalyst of Example 3.

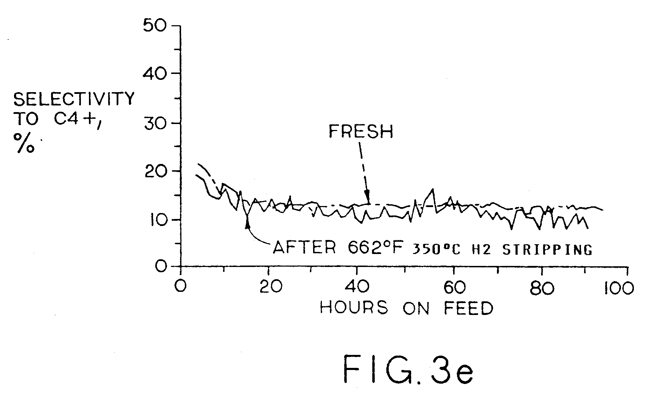

Fig. 3(e) is a graph comparing the selectivity to hydrocarbons having four or more carbon atoms of the fresh and stripped catalyst of Example 3.

### DETAILED DESCRIPTION OF THE INVENTION

Preferably the at least partially deactivated (spent) acetylene hydrogenation catalyst is hydrogen stripped with a mixture of hydrogen and inert gas. When pure hydrogen is not used, the hydrogen concentration in such mixtures may be from 0.1 to less than 100%, preferably from 5 to 10%. Since a stripping gas having only 5-10% of hydrogen may be used during the stripping process, any low value hydrogen source, preferably without carbon monoxide, can be employed. For example, hydrogen may be purchased and mixed fresh with an inert gas, or the hydrogen may come from another refining operation. For example, the overhead effluent from a demethanizer, which contains about 5% H₂ in CH₄ (normally used as fuel gas) may be one potential source for hydrogen. It also may be possible to recycle hydrogen that has already been used to regenerate the catalyst.

The percentage of hydrogen in the gas stripping mixture is not critical to the present invention. In the following examples, a mixture of inert gas and around 9-10% hydrogen was used. Any inert gas may be used in the mixture. The most commonly available and most inexpensive inert gas for use in the invention is nitrogen; therefore, nitrogen is a preferred inert gas for use in the present invention.

Although the following examples of the invention describe regeneration of palladium based acetylene reduction catalysts, one of skill in the art will recognize that the hydrogen stripping process of the present invention may be used to regenerate other types of catalysts that are used to hydrogenate acetylene. For example the present process could be used to regenerate acetylene hydrogenation catalysts which contain: Group VIII metals, such as nickel, cobalt, ruthenium, palladium, and platinum; Group VIB metals, such as chromium, molybdenum, and tungsten; or Group IB metals, such as copper, silver, or gold. However, the preferred catalyst for use in the present invention is a catalyst in which the primary active element is palladium, preferably one where palladium comprises approximately 0.01-0.5% by weight of the catalyst, more preferably 0.02-0.05%. A palladium content higher than about 0.5% could deteriorate at the temperatures used in the process of the present invention. Optionally the palladium may be combined with another metal, such as silver.

The catalyst metal is typically carried on a support such as silica or alumina.

The stripping procedure of the invention takes place at a temperature of from 315-400°C (600-750°F), preferably between about 315-372°C (600-700°F), and more preferably at about 350°C (662°F). In order to avoid deactivating the catalyst, especially a palladium based catalyst, or agglomerating the palladium, the temperature of the hydrogen stripping procedure should not exceed about 400°C. The temperature should be at least 315°C to achieve the substantially complete regeneration which is commercially desirable, and other process conditions will be readily determinable to enable this objective to be reached.

The pressure at which the stripping takes place may not be critical; however, the stripping preferably takes place between atmospheric pressure (0 psig) and 2.07 MPag (300 psig), and more preferably between atmospheric pressure and 345 kPag (50 psig). Lower cost hydrogen sources having a low content of hydrogen typically are more readily available at lower pressures. In the following examples, pressures of about 2.07 MPag and atmospheric pressure were used. Similarly, the time period required for stripping is not critical, save it should be sufficient to achieve substantially complete regeneration. One of skill in the art will recognize that the amount of time needed for the stripping procedure can be determined by monitoring the amount of hydrocarbon present in the catalyst until a minimum plateau level has been achieved. In the following examples, the stripping time ranged between 16-24 hrs.

The flow rate of the hydrogen through the catalyst should be such that the linear velocity through the catalyst should be at least about 15.2 cm/second (0.5 feet/second). This will ensure uniformity of reactant concentrations and temperatures throughout the reactor during regeneration. It has been found that lower velocities may be insufficient to achieve uniform regeneration of the catalyst. The parameter of linear velocity is used herein rather than, e.g., space velocity because linear velocity commonly is used to calculate dispersion and mixing phenomena in packed beds. For example, the axial and radial dispersion of reactant concentration are estimated by calculating Peclet numbers (James Carberry, "Chemical and Catalytic Reaction Engineering", p. 162). The linear velocity also is an important factor in controlling heat transfer in the reactor. Furthermore, linear velocities may differ even where the space velocity remains the same. For example, wide and shallow beds will have lower linear velocities than narrow and deep beds will have. However, narrow and deep beds will have a higher pressure drop than wide and shallow beds. Thus, it is desirable to specify either linear velocity or space velocity with the reactor L/D ratio to ensure proper design or scale up of the reactor.

It is a feature of the invention that substantially complete regeneration is achieved without the need for an additional or associated regeneration step, e.g., hydrocarbon wash or steam treat or, particularly, oxygenation. As shown in Example 1 which follows, the process of the invention enables catalyst to be re-used as fresh catalyst after regular hydrogen only regenerations of the catalytic metal sites, preferably in situ in the acetylene reduction reactor. Nevertheless it may be desirable from time to time to clean carbonaceous deposits from the support (as opposed to the catalyst metal) because hydrogen stripping does not remove all of the carbonaceous deposits from the support. Thus it is possible that an air burn may be desirable eventually in order to clean the support to prevent restriction of flow rate, pressure, etc. However, the number of air burns that may be employed to clean the supports in such a system will be much less than the frequent air burns that heretofore have been required to regenerate the catalyst. An infrequent air burn to clean the support will be much less intrusive and damaging than the frequent, repetitive air burns required in current catalyst regeneration processes.

The following examples illustrates the invention.

### EXAMPLE 1

An aliquot of UCI G58B catalyst (Pd/Al₂O₃), deactivated after 200 hours of use as an acetylene hydrogenation catalyst in olefin stream treatment, was stripped with 10% H₂/Ar at linear velocity greater than 15.2 cm/sec and at a temperature which rose from room temperature to about 750°C (1382°F) at a heating rate of about 13 degrees C/min (23.4°F/min). The carbonaceous residues on the catalyst were analyzed both before and after hydrogen stripping by temperature programmed oxidation (TPO), which detected carbon oxide emission during the TPO burn while the reactor temperature was linearly increased. The TPO spectra of the spent catalyst before and after hydrogen stripping were plotted as Figure 1(a) and (b), respectively.

Before hydrogen stripping, two types of carbonaceous residues were present on the catalyst. A peak at about 400°C (750°F) represents carbonaceous residues that were present on and/or near palladium. A peak at about 510°C (950°F) represents carbonaceous residues that were present on the support. After hydrogen stripping, only one peak appeared at 550°C (1022°F). The presence of only this single peak after hydrogen stripping indicated that the carbonaceous residues that were on and/or near the palladium were removed by the hydrogen stripping. Since acetylene conversion occurs mainly on palladium sites in the catalyst, the removal of carbonaceous residues from the palladium can re-expose the palladium active sites and recover substantially all the activity of the palladium. The hydrocarbons that are stripped off the catalyst can be either recycled to the cracker or used as fuel gas.

### EXAMPLE 2

A BASF HO11 catalyst (Pd/SiO₂) was subjected for 200 hours to the reaction conditions of 1.5% wt C₂H₂ (in an olefinic stream), 1.6 H₂/C₂H₂ molar ratio, 4000 gas hourly space velocity (GHSV), 2.07 MPag (300 psig), and about 66°C (151°F) average bed temperature. After 200 hours, the acetylene conversion of the catalyst decreased from 79% to 58%. The spent catalyst was then regenerated by hydrogen stripping for 16 hours with 5% H₂/N₂ at 2.07 MPag (300 psig) and greater than 15.2 cm/sec linear velocity in the reactor, and about 350°C (662°F). After the hydrogen stripping, the acetylene conversion of the catalyst was 78%. A comparison of the catalyst activity before and after hydrogen stripping is plotted in Figure 2. The results indicate that the hydrogen stripping at about 350°C (662°F) substantially fully regenerated the catalyst.

The temperature used during hydrogen stripping is preferably not too high or else the palladium will begin to agglomerate and will not achieve substantially complete regeneration using only a hydrogen treat step. For example, when the catalyst was further hydrogen stripped at about 420°C (788°F) for 16 hours, under otherwise the same conditions, the activity of the catalyst was reduced to 62% acetylene conversion -- a dramatic decrease in catalyst activity.

### EXAMPLE 3

A spent UCI G58E catalyst (Pd/Ag/Al₂O₃) which had been used for acetylene hydrogenation in an ethylene stream was hydrogen stripped at about 350°C (662°F) and 1 atmosphere pressure with 9% H₂/N₂ at linear velocity in the reactor of greater than 15.2 cm/sec, for 24 hours. The performance of the hydrogen stripped catalyst was then compared to the performance of fresh catalyst at 70 hours into the activity test. The results are tabulated in Table I. The parameters of the activity test carried out in a normal hydrogenation reactor were 1.4% wt C₂H₂, 1.2 H₂/C₂H₂ molar ratio, 4900 GHSV, 2.07 MPag (300 psig), about 102°C (215°F) sand-bath temperature.

**TABLE I**

| | Fresh | H₂ Stripped |
|---|---|---|
| C₂H₂ Conversion | 80% | 80% |
| Selectivity to C₂H₄ | 57% | 67% |
| Selectivity to C₂H₆ | 32% | 23% |
| Selectivity to C₄+ | 11% | 10% |
| Deactivation Rate | -0.1%/hr | -0.2%/hr |

As seen from Table I, the spent catalyst which had been hydrogen stripped according to the invention unexpectedly had a selectivity to ethylene that was 10 points better than the selectivity of the fresh catalyst. Also, the ethane make was substantially reduced after hydrogen stripping. This result suggests that a "de-edging" process may have occurred which reduced the number of palladium sites responsible for over hydrogenating acetylene/ethylene to ethane.

Although the deactivation rate was higher for the hydrogen stripped catalyst, the deactivation rate can be overcome by more frequent hydrogen stripping. Even if more frequent hydrogen stripping is required to regenerate catalyst, the present process may be less time consuming and less damaging to the catalyst than frequent air burns or other additional treatment steps.

## Claims

1. A method of substantially completely regenerating a catalyst that has been used to remove acetylenic impurities from an olefin stream and which has become at least partially deactivated by that use, which comprises substantially stripping the deactivated catalyst of carbonaceous deposits using hydrogen at a linear velocity of at least 15.2 cm/second (0.5 feet per second), at a temperature of from 315 - 400°C (599 - 750°F) and at other conditions such that substantially complete regeneration is effected, wherein said stripping is essentially the only regeneration treatment employed and occurs in the absence of an oxygenation step.

2. The method of claim 1 wherein the temperature is from 315 - 372°C (600 - 700°F), preferably about 350°C (662°F).

3. The method of claim 1 or 2 wherein the hydrogen stripping employs a gas comprising an inert gas, preferably nitrogen, and from 5 to 10% of hydrogen.

4. The method of any preceding claim wherein the catalyst comprises palladium, optionally together with another metal.

5. The method of claim 4 wherein the palladium comprises from 0.01 to 0.5 wt%, preferably from 0.02 to 0.05% of the catalyst.

6. The method of any preceding claim wherein the catalyst has been used to remove acetylenic impurities from ethylene.

7. The method of claim 6 wherein after regeneration the catalyst is used to remove acetylenic impurities from ethylene and the selectivity of the catalyst to ethylene is enhanced.

8. The method of any preceding claim wherein the catalyst comprises a silica or alumina support.

## Patentansprüche

1. Verfahren zur im wesentlichen vollständigen Regenerierung eines Katalysators, der zur Entfernung von acetylenischen Verunreinigungen aus einem Olefinstrom verwendet worden ist und durch diese Verwendung mindestens teilweise desaktiviert worden ist, bei dem der desaktivierte Katalysator unter Verwendung von Wasserstoff mit einer Lineargeschwindigkeit von mindestens 15,2 cm/s (0,5 ft/s) bei einer Temperatur von 315 bis 400°C (599 bis 750°F) und unter solchen weiteren Bedingungen, daß eine im wesentlichen vollständige Regenerierung bewirkt wird, im wesentlichen von kohlenstoffartigen oder kohlenstoffhaltigen Ablagerungen gestrippt wird, wobei das Strippen im wesentlichen die einzige verwendete Regenerierungsbehandlung ist und in Abwesenheit einer Oxygenierungsstufe stattfindet.

2. Verfahren nach Anspruch 1, bei dem bei dem die Temperatur 315 bis 372°C (600 bis 700°F), vorzugsweise etwa 350°C (662°F) beträgt.

3. Verfahren nach Anspruch 1 oder 2, bei dem das Wasserstoffstrippen ein Gas verwendet, das ein Inertgas, vorzugsweise Stickstoff, und 5 bis 10 % Wasserstoff umfaßt.

4. Verfahren nach einem der vorhergehenden Ansprüche, bei dem der Katalysator Palladium, gegebenenfalls zusammen mit einem weiteren Metall, umfaßt.

5. Verfahren nach Anspruch 4, bei dem das Palladium 0,01 bis 0,5 Gew.%, vorzugsweise 0,02 bis 0,05 % des Katalysators umfaßt.

6. Verfahren nach einem der vorhergehenden Ansprüche, bei dem der Katalysator zum Entfernen von acetylenischen Verunreinigungen aus Ethylen verwendet worden ist.

7. Verfahren nach Anspruch 6, bei dem der Katalysator nach der Regenerierung zum Entfernen von acetylenischen Verunreinigungen aus Ethylen verwendet wird und die Selektivität des Katalysators für Ethylen erhöht ist.

8. Verfahren nach einem der vorhergehenden Ansprüche, bei dem der Katalysator einen Siliciumdioxid- oder Aluminiumoxidträger umfaßt.

## Revendications

1. Procédé de régénération pratiquement complète d'un catalyseur qui a été utilisé pour l'élimination d'impuretés acétyléniques d'un courant d'oléfines et a été au moins partiellement désactivé par cette utilisation, qui comprend un dépouillement substantiel du catalyseur désactivé des dépôts carbonés au moyen d'hydrogène à une vitesse linéaire d'au moins 15,2 cm/s (0,5 pied par seconde), à une température de 315 à 400 °C (599 à 750 °F) et dans d'autres conditions telles que soit réalisée une régénération pratiquement complète, ce dépouillement étant essentiellement le seul traitement de régénération employé, et ayant lieu en l'absence d'une opération d'oxygénation.

2. Procédé selon la revendication 1, dans lequel la température est de 315 à 372 °C (600 à 700 °F), de préférence d'environ 350 °C (662 °F).

3. Procédé selon l'une des revendications 1 et 2, dans lequel le dépouillement à l'hydrogène est effectué avec un gaz constitué d'un gaz inerte, de préférence d'azote, et de 5 à 10 % d'hydrogène.

4. Procédé selon l'une des revendications précédentes, dans lequel le catalyseur contient du palladium, facultativement avec un autre métal.

5. Procédé selon la revendication 4, dans lequel le palladium représente de 0,01 à 0,5 % en masse, de préférence de 0,02 à 0,05 % en masse, du catalyseur.

6. Procédé selon l'une des revendications précédentes, dans lequel le catalyseur a été utilisé pour l'élimination d'impuretés acétyléniques de l'éthylène.

7. Procédé selon la revendication 6, dans lequel, après régénération, le catalyseur est utilisé pour l'élimination d'impuretés acétyléniques de l'éthylène et la sélectivité du catalyseur pour l'éthylène est accrue.

8. Procédé selon l'une des revendications précédentes, dans lequel le catalyseur comporte un support en silice ou en alumine.
